(19) Europäisches Patentamt
European Patent Office
Office européen des brevets

(11) **EP 2 253 268 B1**

(12) **EUROPEAN PATENT SPECIFICATION**

(45) Date of publication and mention of the grant of the patent:
**27.08.2014 Bulletin 2014/35**

(51) Int Cl.:
**A61B 5/085** (2006.01)   **A61B 10/00** (2006.01)
**A61B 5/08** (2006.01)

(21) Application number: **09719205.8**

(22) Date of filing: **09.03.2009**

(86) International application number:
**PCT/JP2009/054441**

(87) International publication number:
**WO 2009/113500 (17.09.2009 Gazette 2009/38)**

(54) **RESPIRATION IMPEDANCE MEASURING DEVICE AND METHOD, AND RESPIRATION IMPEDANCE DISPLAY METHOD**

VORRICHTUNG UND VERFAHREN ZUR MESSUNG DES ATEMWIDERSTANDES UND VERFAHREN ZUR ANZEIGE DES ATEMWIDERSTANDES

DISPOSITIF ET PROCÉDÉ DE MESURE D'IMPÉDANCE DE RESPIRATION, ET PROCÉDÉ D'AFFICHAGE D'IMPÉDANCE DE RESPIRATION

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **10.03.2008   JP 2008060002**
**19.06.2008   JP 2008160042**

(43) Date of publication of application:
**24.11.2010   Bulletin 2010/47**

(73) Proprietors:
• **Chest M.I., Incorporated**
**Tokyo 113-0033 (JP)**
• **Tohoku Techno Arch Co., Ltd.**
**Sendai-shi, Miyagi 980-0845 (JP)**

(72) Inventors:
• **KUROSAWA, Hajime**
**Sendai-shi**
**Miyagi 980-8577 (JP)**
• **SHIMIZU, Yoshio**
**Sendai-shi**
**Miyagi 981-3122 (JP)**
• **HOKI, Toshiaki**
**Tokyo 113-0033 (JP)**

(74) Representative: **Kramer - Barske - Schmidtchen**
**Landsberger Strasse 300**
**80687 München (DE)**

(56) References cited:
JP-A- 49 034 185     JP-T- 2007 536 026
US-A- 6 142 952      US-A1- 2005 247 307
US-B1- 6 435 182

• LUTCHEN K R ET AL: "Use Of Transfer Impedance Measurements For Clinical Assessment Of Lung Mechanics", AMERICAN JOURNAL OF RESPIRATORY AND CRITICAL CARE MEDICINE, AMERICAN LUNG ASSOCIATION, NEW YORK, NY, US, vol. 157, 1 January 1998 (1998-01-01), pages 435-446, XP003004877, ISSN: 1073-449X
• IONESCU C ET AL: "Detection of novel respiratory mechanic parameters by means of forced oscillations", PROCEEDINGS OF THE WORLD CONGRESS OF IFAC, XX, XX, no. 16TH, 4 July 2005 (2005-07-04), pages 1-6, XP002604715,
• OOSTVEEN E ET AL: "The forced oscillation technique in clinical practice: methodology, recommendations and future developments", EUROPEAN RESPIRATORY JOURNAL, MUNKSGAARD INTERNATIONAL PUBLISHERS, COPENHAGEN, DK, vol. 22, 1 January 2003 (2003-01-01), pages 1026-1041, XP002551341, ISSN: 0903-1936, DOI: 10.1183/09031936.03.00089403 [retrieved on 2009-10-16]
• TOYOHIRO HIRAI: 'IOS-ho ni yoru Kokyu Teiko no Hyoka' KOKYU TO JUNKAN vol. 51, no. 12, December 2003, pages 1241 - 1244, XP008140628

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 253 268 B1

- **HAJIME KUROSAWA: 'COPD no Shindan to Hai Kino Kensa no Shinpo' KAGAKU RYOHO NO RYOIKI vol. 21, 11 May 2005, pages 74 - 80, 252**

- **HIROMASA OGAWA: 'Kokyu Kino Kensa -Impulse Oscillometry-ho' KOKYU TO JUNKAN vol. 54, no. 6, 15 June 2006, pages 615 - 622, 678, XP008140633**

**EP 2 253 268 B1**

**Description**

TECHNICAL FIELD

The present invention relates to a respiratory impedance measuring apparatus and method that are capable of continuously measuring a respiratory impedance of a human being, etc., and to a respiratory impedance display method.

BACKGROUND ART

Conventionally, an apparatus of this kind is known that includes a sine-wave pressurizing apparatus to apply as a load a sine-wave air vibration pressure to a respiratory system, an air current velocity detector to detect an air current velocity of the respiratory system, an air pressure detector to detect an air pressure of the respiratory system, and a resistance computing unit that calculates breathing resistance from the air current velocity and the air pressure detected by the air current velocity detector and the air pressure detector.

The conventional apparatus: further includes a reference signal converter to convert a signal of the sine-wave air vibration pressure that is applied by the sine-wave pressurizing apparatus into a reference signal and a vector computing device that processes a signal of the air current velocity using the reference signal of the sine-wave air vibration pressure from the reference signal converter and that, thereby, takes out only a component at the same frequency as that of the reference signal; and is adapted to calculate the breathing resistance using the resistance computing unit from the signal of the air current velocity obtained by the vector computing device and the signal of the air pressure detected by the air pressure detector.

As above, this apparatus is adapted to measure the breathing resistance using the resistance computing unit from the signal of the air current velocity obtained by the vector computing device and the signal of the air pressure detected by the air pressure detector and, therefore, noises may be removed even when the amount of ventilation of the breathing is a little and the number of ventilating sessions is large. Therefore the apparatus has an advantage that the apparatus may execute high precision measurement of breathing resistance (see Patent Document 1).

The analysis of the respiratory impedance using pressure oscillations directed to the oral cavity are the subject-matters of various publications, including US 6,142,952 A, US 6,435,182 B1, US 2005/247307 A1, LUTCHEN K R ET AL: "Use Of Transfer Impedance Measurements For Clinical Assessment of Lung Mechanics", AMERICAN JOURNAL OF RESPIRATORY AND CRITICAL CARE MEDICINE, AMERICAN LUNG ASSOCIATION, NEW YORK, NY, US, vol. 157, 1 January 1998 (1998-01-01), pages 435-446, XP003004877, ISSN: 1073-449X, IONESCU C ET AL: "Detection of novel respiratory mechanic parameters by means of forced oscillations", PROCEEDINGS OF THE WORLD CONGRESS OF IFAC, XX, XX, no. 16TH, 4 July 2005 (2005-07-04), pages 1-6, XP002604715, OOSTVEN E ET AL: "The forced oscillation technique in clinical practice: methodology, recommendations and future developments", EUROPEAN RESPIRATORY JOURNAL, MUNKSGAARD INTERNATIONAL PUBLISHERS, COPENHAGEN, DK, vol. 22, 1 January 2003 (2003-01-01), pages 1026-1041, XP002551341, ISSN: 0903-1936, DOI: 10.1183/09031936.03.00089403

However, the removal of the noises is not sufficient even by the conventional apparatus and realization of a higher-perfomance respiratory impedance measuring apparatus is demanded.

Patent Literature 1: Japanese Patent Application the KOKAI Publication No. H03-039140.

DISCLOSURE OF THE INVENTION

Problems to Be Solved by the Invention

The present invention was conceived in view of the current circumstances in respiratory impedance measurement and an object thereof is to provide a respiratory impedance measuring apparatus and method that are capable of continuously measuring impedance for a plurality of frequencies at one time. Another object thereof is to provide a respiratory impedance measuring apparatus and method that are capable of removing noises and measuring respiratory impedance with extremely high precision, and a respiratory impedance display method.

Means for Solving the Problems

The objects indicated above are achieved by a respiratory impedance measuring apparatus according to claim 1 and a respiratory impedance measurement method according to claim 7. Further developments of the invention are given in the dependent claims.

The respiratory impedance measuring apparatus according to the present invention characteristically includes:

3

a pressurizing means to apply an air vibration pressure to the inside of an oral cavity, a control means that causes the air vibration pressure to be generated, by an oscillation wave that is a signal to drive this pressurizing means and that is a signal obtained by frequency-culling executed such that the signal has only the frequency components that are left after the culling is executed from a plurality of different frequencies; a pressure detecting means that detects the pressure of the inside of an oral cavity; a flow detecting means that detects a flow generated by breathing; a Fourier transforming means that obtains signals obtained by the pressure detecting means and the flow detecting means under the pressurized condition provided by the pressurizing means, that Fourier-transforms the signals obtained, and that obtains a spectrum; an extracting means that obtains a breathing high frequency component based on a spectrum that corresponds to the frequency component culled from the result of the transformation by the Fourier transforming means, and that takes out an oscillation wave component by subtracting the breathing high frequency component from a spectrum that corresponds to a frequency component left by the culling; and a computing means that divides a pressure component by a flow component for each frequency for the result of the extraction by the extracting means.

The respiratory impedance measuring apparatus according to the present invention is **characterized in that** the control means causes the air vibration pressure to be generated, by the oscillation wave having only $n/T$ (n: an integer, T: a real number) frequency components, by giving a pulse wave having the cycle T as the frequency culling. Such frequency-culling is referred to as <frequency-culling 1>. In <frequency-culling 1>, when T is determined, a plurality of frequency components are obtained that are left by culling the frequency components other than $n/T$ (n: an integer) frequency components.

The respiratory impedance measuring apparatus according to the present invention is **characterized in that** the control means combines a plurality of sine waves at different frequencies, thereby, obtains an oscillation wave frequency-culled, and causes the air vibration pressure by the oscillation wave to be generated. Such frequency-culling is referred to as <frequency-culling 2>. In <frequency-culling 2>, the signal is also enabled to have only a plurality of integer-frequency components left by culling desired integers from consecutive integers and, therefore, the signal may include a plurality of integer-frequency components left by culling odd-number frequency components.

The respiratory impedance measuring apparatus according to the present invention is **characterized in that** the control means includes a signal input means that supplies an input signal to the pressurizing means such that an oscillation wave having a desired pressure waveform is an output signal, based on reverse computation using the input signal and the output signal of the pressurizing means and a transfer function of the pressurizing means.

The respiratory impedance measuring apparatus according to the present invention is **characterized in that** the signal input means supplies to the pressurizing means as an input signal a signal obtained by adding a specific value to each of frequency components of the signal obtained by the reverse computing, or by reverse computing the signal formed by adding an impulse to an onset portion of the output signal.

The respiratory impedance measurement method according to the present invention characteristically includes: a pressurizing step to apply an air vibration pressure to the inside of an oral cavity; a control step of causing the air vibration pressure to be generated, by an oscillation wave that is a signal to control this pressurizing step and that is a signal obtained by frequency-culling executed such that this signal has only the frequency component that is left after the culling is executed from a plurality of different frequencies; a pressure detecting step of detecting the pressure of the inside of the oral cavity; a flow detecting step of detecting the flow generated by breathing; a Fourier-transforming step of obtaining signals obtained at the pressure detecting step and the flow detecting step under the pressurized condition provided at the pressurizing step, Fourier-transforming the signals obtained, and, thereby, obtaining a spectrum; an extracting step of obtaining a breathing high frequency component based on a spectrum that corresponds to the frequency components culled from the result of the transformation at the Fourier transforming step, and taking out an oscillation wave component by subtracting the breathing high frequency component from a spectrum that corresponds to frequency components left by the culling; and a computing step of dividing a pressure component by a flow component for each of frequencies for the result of the extraction at the extracting step, and the method is **characterized in that** each of the steps is executed by processing and control of a computer.

The respiratory impedance measurement method according to the present invention is **characterized in that**, at the control step, the air vibration pressure is caused to be generated, by an oscillation wave having only $n/T$ (n: an integer, T: a real number) frequency components by supplying a pulse wave having a cycle T as the frequency-culling. Such frequency-culling is <frequency-culling 1>.

The respiratory impedance measurement method according to the present invention is **characterized in that**, at the control step, the air vibration pressure is caused to be generated, by an oscillation wave by obtaining the oscillation wave frequency-culled, that is obtained by combining a plurality of sine waves at a plurality of different frequencies. Such frequency-culling is <frequency-culling 2>.

The respiratory impedance measurement method according to the present invention is **characterized in that** the control step includes a signal input step of supplying an input signal at the pressurizing step such that an oscillation wave having a desired pressure waveform is an output signal, based on reverse computation using the input signal and the output signal at the pressurizing step and a transfer function at the pressurizing step.

The respiratory impedance measurement method according to the present invention is **characterized in that**, at the signal input step, a signal is supplied at the pressurizing step, that is obtained by adding a specific value to each of frequency components of the signal obtained by the reverse computing, or by reverse computing the signal formed by adding an impulse to an onset portion of the output signal.

The respiratory impedance display method according to the present invention is **characterized in that**, in a respiratory impedance display method of executing display on a displaying apparatus based on the respiratory impedance measured by the respiratory impedance measuring apparatus: the display is executed by three-dimensionally taking values based on an impedance axis, a frequency axis, and a time axis; an image is created by including respiratory impedance obtained by executing an interpolation process for the culled frequencies, in the display to execute the display by three-dimensionally taking values; and, thereby, the display is executed.

The respiratory impedance display method according to the present invention is **characterized in that** the display is executed by creating the image with the length in the direction of the time axis, that is taken to be a length enough to repeat therein at least two sets of exhalation and inhalation.

The respiratory impedance display method according to the present invention is **characterized in that** the display is executed by creating an image that expresses magnitude of an impedance value using variation in color or variation in gradation.

Effects of the Invention

According to the present invention: an air vibration pressure by an oscillation wave that is frequency-culled is applied to the inside of an oral cavity; the pressure of the inside of the oral cavity is detected; the flow of breathing is detected; a spectrum is obtained by Fourier-transforming these signals obtained; a breathing high frequency component that contributes as a noise is obtained using a spectrum that corresponds to frequency components culled from the result of the Fourier transformation; the breathing high frequency component is subtracted from a spectrum that corresponds to the frequency components left by the culling; thereby, an oscillation wave component is extracted; computing is executed of dividing a pressure component by a flow component for each of frequencies for the result of this extraction; and, thereby, respiratory impedance is obtained. Therefore, the respiratory impedance may be obtained using the oscillation wave component from which the breathing high frequency component is securely removed and, therefore, impedance measurement with extremely high precision may be enabled.

According to the present invention: the air vibration pressure by the oscillation wave having only the n/T (n: an integer, T: a real number) frequency components is caused to be generated by supplying the pulse having the cycle T; therefore, the breathing high frequency component is obtained using the spectrum that corresponds to the frequency components culled; and the breathing high frequency component is subtracted from the spectrum that corresponds to the frequency components left by the culling. Therefore, the breathing high frequency component may securely be removed and the respiratory impedance measurement with extremely high precision is enabled.

According to the present invention, the plurality of sine waves at the plurality of different frequencies are combined and, thereby, the air vibration pressure by the oscillation wave that is frequency-component-culled is caused to be generated. Therefore, only the breathing high frequency component is included by the spectrum that corresponds to the frequency components culled and, therefore, the breathing high frequency component may securely be removed and the respiratory impedance measurement with extremely high precision is enabled.

According to the present invention, an input signal is supplied to a pressurizing executing portion such that the oscillation wave having a desired pressure waveform is the output signal based on the reverse computation using the input signal and the output signal for the pressurizing and a transfer function of the pressurizing executing portion. Therefore, the measurement may be executed using the oscillation wave having the desired pressure waveform and respiratory impedance measurement with extremely high precision is enabled.

According to the present invention, the input signal is the signal obtained by adding a specific value to each of the frequency components of the signal obtained by the reverse computing, or by reverse computing the signal formed by adding an impulse to the onset portion of the output signal. Therefore, the signal waveform of the result of the reverse computing may be stabilized and, thereby, the measurement using the oscillation wave having a desired waveform may be executed and the respiratory impedance measurement with extremely high precision is enabled.

According to the respiratory impedance display method according to the present invention, in the respiratory impedance display method of executing display on a displaying apparatus based on the respiratory impedance measured by the respiratory impedance measuring apparatus: the display is executed three-dimensionally taking values based on the impedance axis, the frequency axis, and the time axis; an image is created by including the respiratory impedance obtained by executing an interpolation process for the culled frequencies, in the display to execute display three-dimensionally taking values; and, thereby, the display is executed. Therefore, the result of the interpolation process is also displayed as an image. Therefore, variation of the impedance value may minutely and smoothly be displayed and grasp of the impedance for the whole frequencies may properly be executed.

According to the respiratory impedance display method according to the present invention, the display is executed by creating the image with the length in the direction of the time axis that is a length long enough to repeat therein at least two sets of exhalation and inhalation. Therefore, not an observation of a sudden variation but an observation having a specific span is enabled and, thereby, proper observations may be secured.

According to the respiratory impedance display method according to the present invention, the display is executed by creating the image that expresses magnitude of an impedance value using variation in color or variation in gradation. Therefore, to obviously distinguish the magnitudes of impedance values is easily enabled and it is expected that the method is very useful for various researches and inspections that use the respiratory impedance.

BEST MODES FOR CARRYING OUT THE INVENTION

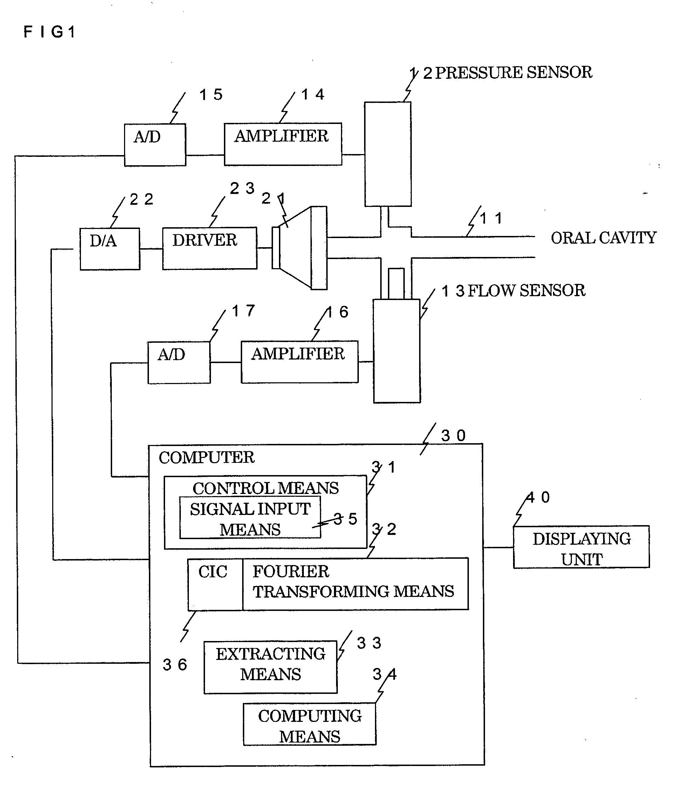

Embodiments of a respiratory impedance measuring apparatus and method according to the present invention will be described with reference to the accompanying drawings. Fig. 1 is a diagram of the configuration of the embodiment of the respiratory impedance measuring apparatus according to the present invention. The respiratory impedance measuring apparatus includes as its main components: a tube 11 whose tip is attached to an oral cavity of a human and through which a breathing flow flows; a pressure sensor 12 that is attached to the tube 11 and that makes up a pressure detecting means to detect the pressure in the oral cavity; a flow sensor 13 that makes up a flow detecting means of detecting the flow of breathing at the same position as that of the pressure sensor 12; a loudspeaker 21 that makes up a pressurizing means to apply an air vibration pressure to the inside of the oral cavity; and a computer 30.

An output signal of the pressure sensor 12 is amplified by an amplifier 14, is digitized by an A/D converter 15, and is taken in by the computer 30. An output signal of the flow sensor 13 is amplified by an amplifier 16, is digitized by an A/D converter 17, and is taken in by the computer 30.

The computer 30 includes a control means 31, a Fourier transforming means 32, an extracting means 33, and a computing means 34. The control means 31 includes a signal input means 35. The control means 31 outputs a signal driving the loudspeaker 21 that is the pressurizing means and causes the air vibration pressure by an oscillation wave having only odd-number frequency components or even-number frequency components, to be generated. An output of the control means 31 is converted into an analog signal by a D/A converter 22 and is sent to a driver 23. The driver 23 drives the loudspeaker 21 and, thereby, the air vibration pressure is applied to the inside of the oral cavity.

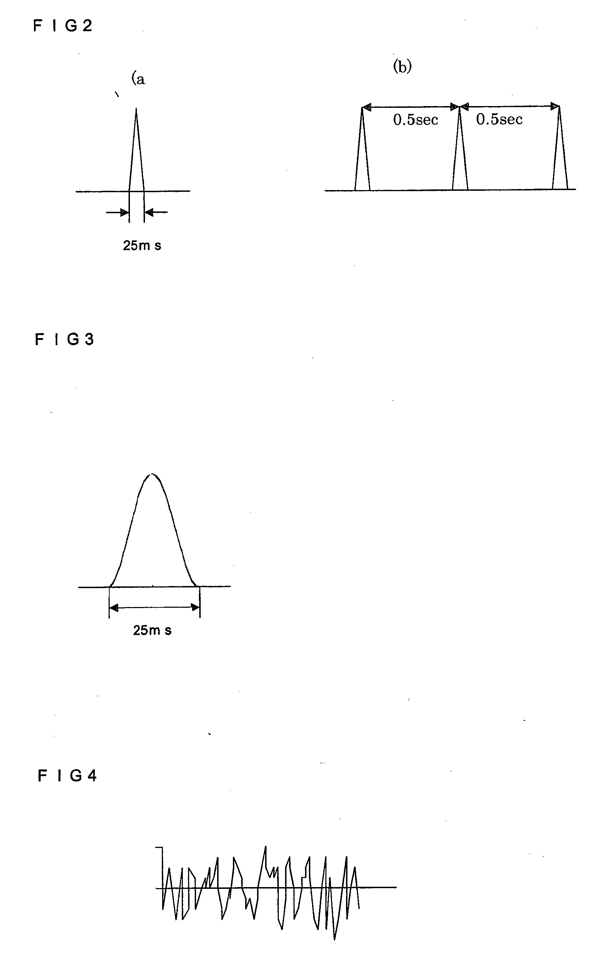

In the above, the control means 31 causes the air vibration pressure by the oscillation wave having n/T (n: an integer, T: a real number) frequency components, to be generated by giving a pulse wave having the cycle of T second (<frequency-culling 1>). Though various waveforms may be considered as the pulse wave, for example, as depicted in Fig. 2 (a), a triangular pulse has the temporal width of about 25 ms at the base level. When this triangular pulse is output with the cycle T that is, for example, T=0.5 second, a triangular pulse wave having a spectrum of 2, 4, 6, 8 Hz, ... may be given (Fig. 2(b)). When the triangular pulse is output with the cycle T that is, for example, T=0.333 second, a triangular pulse wave having a spectrum of 3, 6, 9, 12 Hz, ... may be given.

As depicted in Fig. 3, a Hanning pulse as another example has the temporal width of about 25 ms at the base level. A pulse wave using this pulse is created and output similarly to the case of the triangular pulse wave.

The control means 31 causes the air vibration pressure by an oscillation wave having only desired real-number frequency components, to be generated by giving a wave obtained by combining a plurality of sine waves at a plurality of different frequencies (<frequency-culling 2>). In this case, a signal depicted in Fig. 4 that is a noise wave is output. In this case, a noise wave having only even-number frequency components is obtained by combining sine waves having even-number frequencies such as, for example, 2, 4, 6, ..., 34 Hz. A noise wave having only odd-number frequency components is obtained by combining sine waves having odd-number frequencies such as, for example, 1, 3, 5, ..., 33 Hz. A noise is realized by randomizing the phase of each the sine waves.

The signal input means 35 included in the control means 31 supplies an input signal to the loudspeaker 21 such that an oscillation wave having a desired waveform is an output signal, based on reverse computing using an input signal and an output signal of the loudspeaker 21, and a transfer function of the loudspeaker 21.

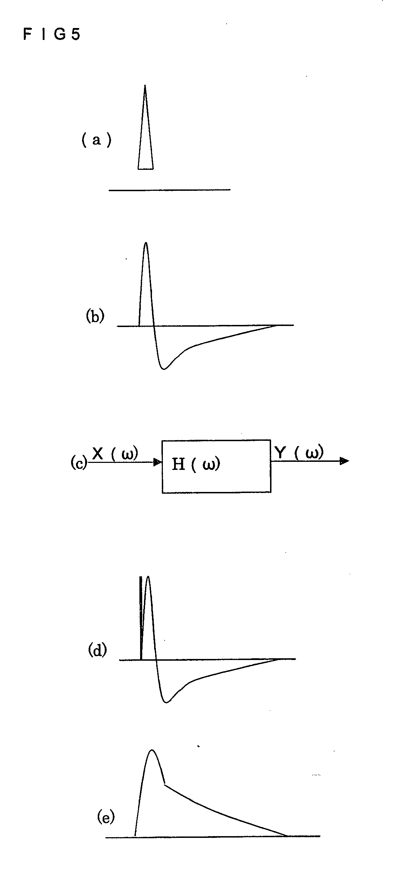

More specifically, describing using, for example, a triangular pulse, when the loudspeaker 21 is driven by inputting thereinto a triangular pulse as depicted in Fig. 5 (a), an output signal of the loudspeaker 21 becomes a signal having a local maximum point on each of the upper and the lower sides of the zero level as depicted in Fig. 5 (b) . A model as depicted in Fig. 5 (c) is considered. Representing the transfer function of the loudspeaker 21 as "$H(\omega)$", the input signal thereof as "$X(\omega)$", and the output signal thereof as "$Y(\omega)$", the following holds and, therefore, x'(t) is obtained by reverse computation and is used as a driving signal.

[Eq. 1]

$$Y(\omega) = X(\omega) H(\omega)$$

Representing an input as X'((ω)) with which X(ω) is obtained,

$$X(\omega) = X'(\omega) H(\omega)$$

$$X'(\omega) = \frac{X(\omega)}{H(\omega)} = \frac{X(\omega)}{\dfrac{Y(\omega)}{X(\omega)}} = \frac{X^2(\omega)}{Y(\omega)}$$

$$x'(t) = F^{-1}(X'(\omega)) \quad \dots \quad (\text{Equation 1})$$

Practically,

$$x'(t) = F^{-1}\left(\frac{X^2(\omega)}{Y(\omega) + A_0}\right) \quad \dots \quad (\text{Equation 2})$$

$Y(\omega)$ obtained has no component that includes frequencies up to a high frequency and, therefore, x'(t) obtained from (Equation 1) is unstable. Therefore, as expressed in (Equation 2), a term obtained by adding a constant "$A_0$" to the denominator of $X'(\omega)$ is inversely Fourier-transformed and, thereby, x' (t) is obtained and is used as the driving signal. The signal x' (t) depicted in Fig. 5 (e) may also be obtained by reverse-computing a signal formed by adding an impulse to an onset portion as depicted in Fig. 5(d) of an output signal of the loudspeaker 21 as depicted in Fig. 5(b).

Though the case for the triangular pulse is described in the above, as to a Hanning pulse, a signal may also be obtained by the reverse computation and this signal may also drive the loudspeaker 21. As to a sine wave, a signal may also be obtained by the reverse computation and a noise wave may also be obtained by this signal combining.

As to which one of the pulse wave, the noise wave, and the sine wave having a single frequency is used, an instruction may be given to the computer 30 using a keyboard, etc., not depicted and, in response to this, the control means 31 outputs a signal waveform selected thereby.

The Fourier transforming means 32, the extracting means 33, and the computing means 34 included in the computer 30 will be described. Under the pressurized condition in the oral cavity caused by a driving of the loudspeaker 21 as above, the Fourier transforming means 32 obtains signals using the pressure sensor 12 and the flow sensor 13, Fourier-transforms these signals obtained, and obtains a spectrum. A CIC filter 36 is provided in the pre-stage of the Fourier transforming means 32 and separates a breathing signal and an oscillation component obtained by the pressure sensor 12 and the flow sensor 13 from each other. The Fourier transforming means 32 takes out a signal using a Hanning window before the processing when necessary.

The extracting means 33 obtains a breathing high frequency component using the spectrum that corresponds to the frequency components culled from the result of the transformation by the Fourier transforming means 32, and takes out the oscillation wave component by subtracting the breathing high frequency component from the spectrum that corresponds to the frequency components left by the culling. Describing based on <frequency-culling 1>, as to the spectrum obtained by the Fourier transforming means 32, the breathing high frequency component is obtained using the spectrum that corresponds to other frequencies excluding n/T (n: an integer) frequency components, and the oscillation wave component is taken out by subtracting the breathing high frequency component from the spectrum that corresponds to the frequency components left by the culling (n/T-frequency components).

Describing based on <frequency-culling 2>, as to the spectrum obtained by the Fourier transforming means 32, the extracting means 33 obtains the breathing high frequency component using the spectrum that corresponds to the frequency components (odd-number frequency components or even-number frequency components) that are different from the frequency components (in this case, even-number frequency components or odd-number frequency components) given to the loudspeaker 21, and takes out the oscillation wave component by subtracting the breathing high frequency component from the spectrum that corresponds to the frequency components given to the loudspeaker 21.

As to the result of the extraction by the extracting means 33, the computing means 34 calculates respiratory impedance by dividing a pressure component by a flow component for each frequency. Representing the respiratory impedance as Z(ω), an oscillation wave component of the pressure in the oral cavity as P(ω), and an oscillation wave component of the flow as F(ω) and assuming that the respiratory impedance Z(ω) includes a resistance component R(ω) and a reactance component X(ω), the respiratory impedance Z(ω) is obtained using the following equations.

[Eq. 2]

$$Z(\omega) = \frac{P(\omega)}{F(\omega)} = R + j\left(\omega L - \frac{1}{\omega C}\right) = R(\omega) + jX(\omega) \quad \dots \text{(Equation 3)}$$

The respiratory impedance Z(ω) obtained by the computing means 34 is converted into a display signal for a displaying unit 40 such as an LCD that is connected to the computer 30 and is output to the displaying unit 40 and, thereby, display is executed.

Operations by the respiratory impedance measuring apparatus configured as above will be described. In this example, the triangular pulse wave is selected and a measuring operation is started. The loudspeaker 21 is driven with the cycle of T second (for example, at intervals of 0.5 second) by the control means 31 and the signal input means 35 using the waveform obtained by the reverse computation.

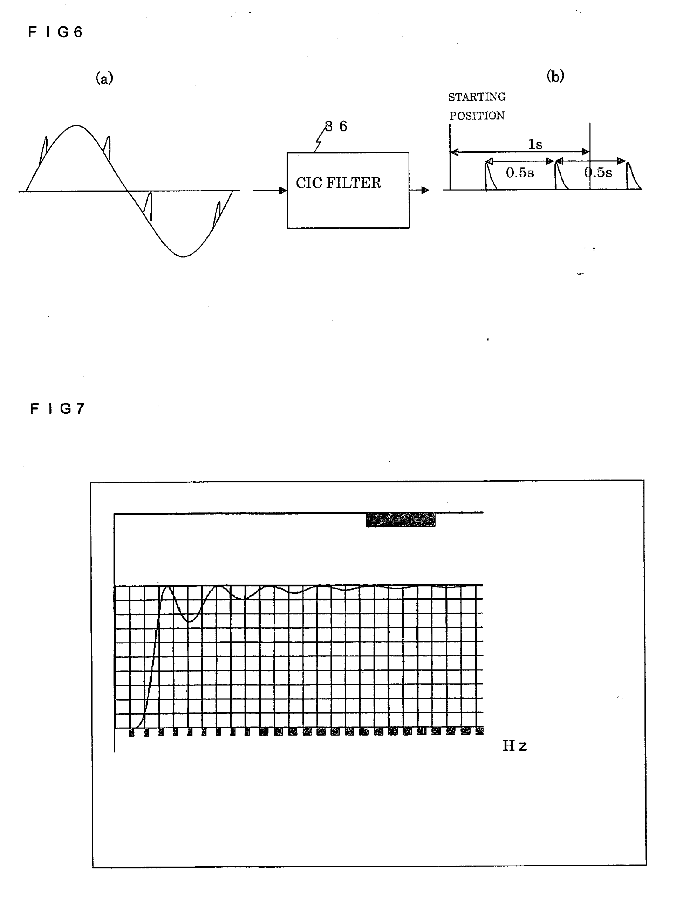

At this time, both of the waveforms of the signals obtained by the pressure sensor 12 and the flow sensor 13 each are a waveform formed by superimposing the triangular pulse wave on the breathing signal as depicted in Fig. 6(a). This waveform is passed through the CIC filter 36 and the separation of the breathing wave and the oscillation wave (the triangular pulse wave) from each other is executed. Fig. 7 depicts the frequency property of the CIC filter 36. The CIC filter 36 may execute the separation without any shift of the phase. However, the breathing signal includes a high frequency component (the same frequency band as that of the oscillation signal) and, therefore, the separation may not be completely executed.

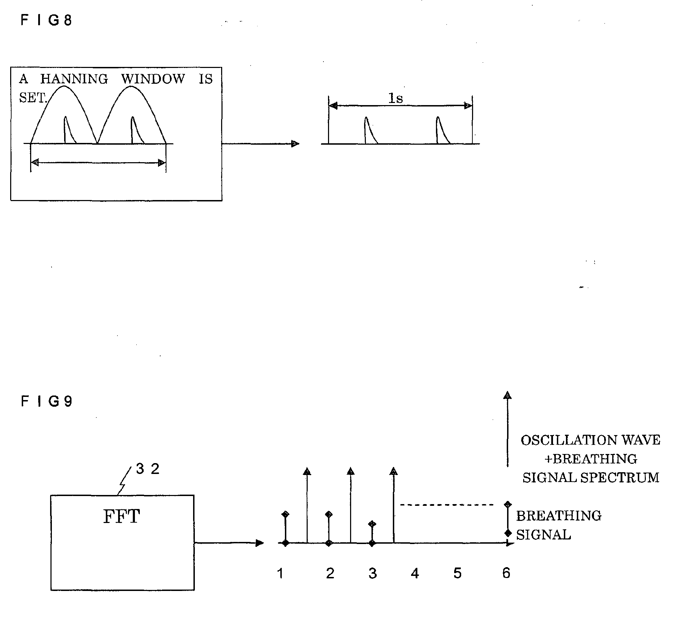

After the separation by the CIC filter 36, as depicted in Fig. 6 (b), as to the oscillation wave, a section of one second is taken out at the intermediate point between two triangular pulses and is used for signal processing. As depicted in Fig. 8, a section of T second is taken out and a process using a Hanning window is executed for each pulse and, thereby, pulses are taken out.

Following the process using the Hanning window, Fourier transformation by the Fourier transforming means 32 is executed and a spectrum is obtained. At this time, as to the spectrum obtained, for example, when a pulse is driven with the cycle of 0.5 second, as depicted in Fig. 9, the breathing signal spectrum is obtained that includes no oscillation wave component in its spectrum of odd-number frequencies of 1, 3, 5, ... that correspond to the frequency components culled. The spectrum of even-number frequencies of 2, 4, 6, ... that corresponds to the frequency components left by the culling includes the oscillation wave component and the breathing signal component.

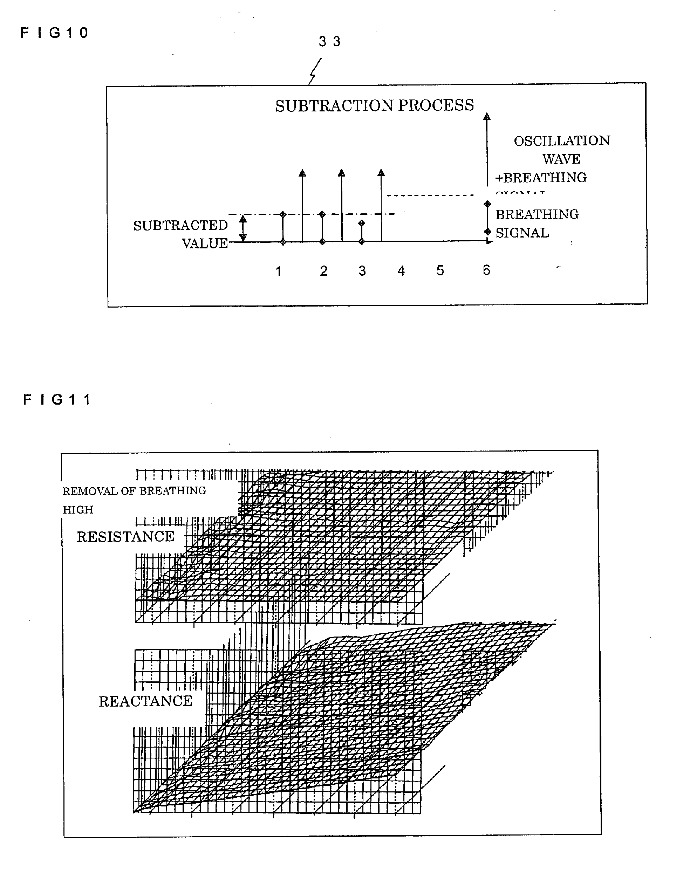

As depicted in Fig. 10, the extracting means 33 subtracts a noise component that is estimated from the spectrum of the odd-number frequencies, from the spectrum of the even-number frequencies and, thereby, takes out the oscillation wave component.

The breathing high frequency signal that is equal to or higher than 3 Hz and that is conventionally considered not to be included in the breathing signal, is removed by the processing of the extracting means 33 and, therefore, high precision respiratory impedance measurement is enabled. The computing means 34 divides the pressure component by the flow component and, thereby, calculates the respiratory impedance as expressed by Equation (2) for each frequency for the result of the extraction by the extracting means 33. A display signal of the respiratory impedance calculated is created and is output to the displaying unit 40 and, thereby, display is executed.

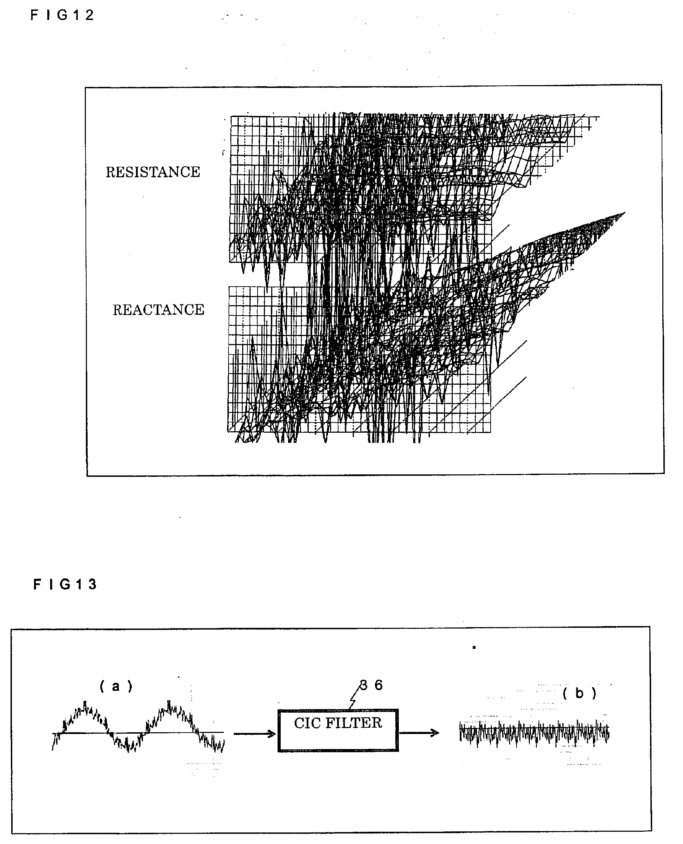

The respiratory impedance that is measured and displayed as above is depicted in Fig. 11. Fig. 12 depicts the respiratory impedance obtained when the breathing high frequency the removal of the breathing high frequency signal is not executed. In each of Figs. 11 and 12, the axis of abscissa is a frequency axis whose one section of graduation corresponds to 1 Hz and the axis of ordinate represents the impedance. An oblique axis is the time axis. A genuine resistance portion is displayed in the upper portion of the diagram and a reactance portion is displayed in the lower portion of the diagram. In this case, by consecutively giving the triangular pulses at intervals of 0.5 second, display of new impedance appears one after another and the display is updated. Thereby, continuous measurement of the impedance is executed. As apparently seen from Figs. 11 and 12, it is understood that the noise is removed and high precision respiratory impedance measurement is enabled. As apparent from the subtracting process by the extracting means 33, the component left by the subtraction is the even-number-frequency component of 2, 4, 6, ... that corresponds to the frequency component left by the culling and, the odd-number-frequency component of 1, 3, 5, ... that corresponds to the frequency component culled is not present. The computing means 34 executes an interpolating process and, thereby, respiratory impedance measurement is enabled for the component that is not present.

The case where the noise wave is selected instead of the triangular pulse wave and the measuring operation is started (<frequency-culling 2>) will be described. The loudspeaker 21 is driven by the control means 31 and the signal input means 35 using the noise wave having only even-number-frequency component based on the waveform obtained by the combining. At this time, both of the waveforms of the signals obtained by the pressure sensor 12 and the flow sensor 13 each are a waveform formed by superimposing the noise wave on the breathing signal as depicted in Fig. 13(a). These signals are passed through the CIC filter 36 and, thereby, the separation of the breathing wave and the oscillation wave (noise wave) from each other is executed (Fig. 13(b)).

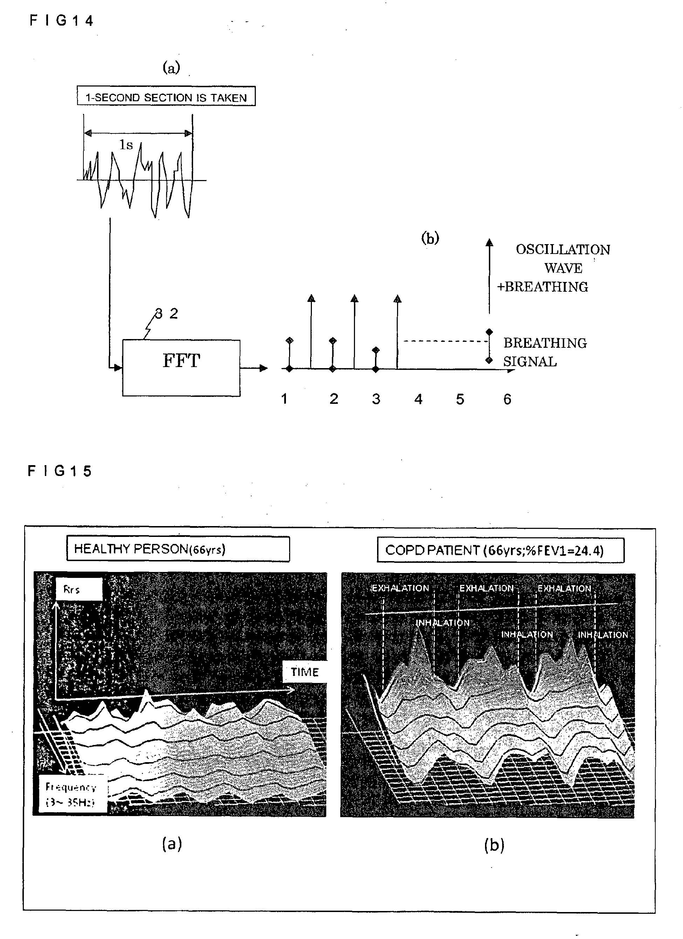

After the separation by the CIC filter 36, as depicted in Fig. 14 (a), as to the oscillation wave, a section of one second is taken out and is used for the signal processing. As depicted in Fig. 14(b), Fourier transformation by the Fourier transforming means 32 is executed for the noise wave from which the section of one second is taken out and, thereby, the spectrum is obtained.

As to the spectrum obtained by the Fourier transformation, the loudspeaker 21 is driven by the noise wave having only the even-number-frequency component combined by the control means 31 and the signal input means 35. Therefore, a breathing signal spectrum is obtained whose spectrum of the odd-number frequencies of 1, 3, 5, ... that corresponds to the frequency components culled does not include the oscillation wave component. The spectrum of the even-number frequencies of 2, 4, 6, ... that corresponds to the frequency components left by the culling includes the oscillation wave component and the breathing signal component.

The extracting means 33 executes subtraction of the odd-number-frequency spectrum from the even-number-frequency spectrum and, thereby, takes out the oscillation wave component. The processes after this are same as the processes executed when the triangular pulse wave is used (Figs. 9 and 10 and the computation using Equation (2)). A display signal of the respiratory impedance calculated is created and is output to the displaying unit 40, and, thereby, display is executed. When the noise wave is used as above, the noise may also be removed and high precision respiratory impedance measurement is enabled. By continuously giving the noise wave, new impedance display appears one after another and, thereby, the display is updated. Thereby, continuous measurement of the impedance is executed. When the noise wave is used, display whose noise is removed is executed similarly to the case depicted in Fig. 11 and the continuous measurement of the impedance is enabled.

In the embodiment of the present invention, the computing means 34 creates the image to execute the display on the displaying apparatus and executes the display and, thereby, a respiratory impedance display method is realized. As to the respiratory impedance calculated by the computing means 34, the computing means 34, for example: determines a coordinate such that each frequency value is taken from the side distant from a viewer to the side close thereto of the image; takes out a resistance component Rrs for each frequency; plots this in the direction of the height of the screen of the displaying apparatus; takes the measurement time in the rightward direction of the screen; creates a three-dimensional image as depicted in Fig. 15; and displays the three-dimensional image on the displaying apparatus. This display is executed three-dimensionally taking values using the impedance axis, the frequency axis, and the time axis.

For creating the image, an image is created and is displayed that is formed by including the respiratory impedance obtained by executing the interpolation process for the frequencies culled, in the case where values are three-dimensionally taken. For example, when the odd-number frequencies are culled, two impedance values are already obtained that correspond to even-number frequencies that are adjacent to an odd number culled. Therefore, the average of these two impedance values is obtained and is used as an impedance value that corresponds to the frequency culled. In this manner, the result of the interpolation process is also converted into an image and is displayed as the image and, therefore, variation of the impedance value may minutely and smoothly be displayed and grasp of the impedance for the whole frequencies may properly be executed.

The sampling time is 0.5 second and, as depicted in Fig. 15(b), the image is created and displayed taking the length in the direction of the time axis that is a length long enough to repeat therein at least two sets of exhalation and inhalation. In the example of Fig. 15, the length is taken to be long enough to repeat therein three sets of exhalation and inhalation.

An image is also created and displayed that expresses the magnitude of the impedance value using variation in color or variation in gradation. In Fig. 15, an image is created and displayed being colored for the resistance value Rrs based on the color scale presented in the lower portion of Fig. 15.

Images obtained by the processes are displayed and, therefore, a subject only has to repeat inhaling and exhaling and images as depicted in Fig. 15 may automatically and time-sequentially be created and displayed. Each of the images may visually be observed as an image that expresses the variation of the respiratory impedance using variation in color or variation in gradation, including the portions that correspond to the frequencies culled.

Therefore, as apparently seen from the variation of the respiratory impedance of a 66-year-old healthy person depicted in Fig. 15(a) and the variation of the respiratory impedance of a 65-year-old COPD (chronic obstructive pulmonary disease) patient depicted in Fig. 15(b), to visually and obviously distinguish a non-healthy person and a healthy person from each other is easily enabled and it is expected that the method is very useful for various researches and inspections that use respiratory impedance. "%FEV1" of Fig. 15(b) is a value that indicates how many % of the forced vital capacity is exhaled in one second. Therefore, in this example, it is presented that 24.4% was able to be exhaled in one second.

BRIEF DESCRIPTION OF DRAWINGS

[Fig. 1] Fig. 1 is a diagram of the configuration of a respiratory impedance measuring apparatus according to an embodiment of the present invention.

[Fig. 2] Fig. 2 is a diagram of an example of a triangular pulse wave that is an oscillation wave used in the respiratory impedance measuring apparatus according to the embodiment of the present invention.

[Fig. 3] Fig. 3 is a diagram of an example of a Hanning pulse wave that is an oscillation wave used in the respiratory impedance measuring apparatus according to the embodiment of the present invention.

[Fig. 4] Fig. 4 is a diagram of an example of a noise wave that is an oscillation wave used in the respiratory impedance measuring apparatus according to the embodiment of the present invention.

[Fig. 5] Fig. 5 is a diagram for explaining the process of creating, using reverse computation, the oscillation wave used in the respiratory impedance measuring apparatus according to the embodiment of the present invention.

[Fig. 6] Fig. 6 is a diagram of a process of obtaining the respiratory impedance using the triangular pulse wave that is the oscillation wave by the respiratory impedance measuring apparatus according to the embodiment of the present invention.

[Fig. 7] Fig. 7 is a diagram of the frequency property of a filter employed in the respiratory impedance measuring apparatus according to the embodiment of the present invention.

[Fig. 8] Fig. 8 is a diagram of a process of obtaining the respiratory impedance using the triangular pulse wave that is the oscillation wave by the respiratory impedance measuring apparatus according to the embodiment of the present invention.

[Fig. 9] Fig. 9 is a diagram of a process of obtaining the respiratory impedance using the triangular pulse wave that is the oscillation wave by the respiratory impedance measuring apparatus according to the embodiment of the present invention.

[Fig. 10] Fig. 10 is a diagram of a process of obtaining the respiratory impedance using the triangular pulse wave that is the oscillation wave by the respiratory impedance measuring apparatus according to the embodiment of the present invention.

[Fig. 11] Fig. 11 is a diagram of the respiratory impedance obtained by the respiratory impedance measuring apparatus according to the embodiment of the present invention.

[Fig. 12] Fig. 12 is a diagram of respiratory impedance obtained by a respiratory impedance measuring apparatus that does not use the approach of the present invention.

[Fig. 13] Fig. 13 is a diagram of a process of obtaining the respiratory impedance using a noise wave that is the oscillation wave by the respiratory impedance measuring apparatus according to the embodiment of the present invention.

[Fig. 14] Fig. 14 is a diagram of a process of obtaining the respiratory impedance using a noise wave that is the oscillation wave by the respiratory impedance measuring apparatus according to the embodiment of the present invention.

[Fig. 15] Fig. 15 is a diagram of an example of respiratory impedance for each of a healthy person and a non-healthy person displayed using the respiratory impedance measuring apparatus according to the embodiment of the present invention. Explanations of Letters or Numerals

11  tube
12  pressure sensor
13  flow sensor
21  loudspeaker
30  computer
31  control means

32    Fourier transforming means
33    extracting means
34    computing means
35    signal input means
36    CIC filter
40    displaying unit

**Claims**

1.  A respiratory impedance measuring apparatus comprising:

    a pressurizing means (21) that is configured to apply an air vibration pressure to an inside of an oral cavity;
    a control means (30) that is configured to cause the air vibration pressure to be generated by an oscillation wave, wherein the oscillation wave is a signal that drives the pressurizing means (21);
    a pressure detecting means (12) that is configured to detect a pressure of the inside of the oral cavity;
    a flow detecting means (13) that is configured to detect a flow generated by breathing;
    a Fourier transforming means (32) that is configured to obtain and Fourier-transform signals obtained by the pressure detecting means (12) and the flow detecting means (13) under a pressurized condition provided by the pressurizing means (21), thereby obtaining as a result a pressure spectrum for the signal obtained by the pressure detecting means (12) and a flow spectrum for the signal obtained by the flow detecting means (13);
    an extracting means (33); and
    a computing means (34) that is configured to divide a pressure oscillation wave component by a flow oscillation wave component; **characterised in that** :

    the oscillation wave is obtained by frequency-culling, and the frequency-culling is configured such that the signal has only those frequency components from a plurality of different frequencies that are left after the culling is executed; and;
    the extracting means is configured to obtain, for each of the pressure spectrum and the flow spectrum, a breathing high frequency component based on those frequency components of the pressure spectrum and the flow spectrum, respectively, that were culled during the frequency-culling,
    to obtain a culled pressure spectrum and a culled flow spectrum, each comprising frequency components of the pressure spectrum and the flow spectrum, respectively, that were left by the frequency-culling, and
    to extract pressure oscillation wave components and flow oscillation wave components by subtracting the breathing high frequency component from the culled pressure spectrum and the culled flow spectrum, respectively; and the division of a pressure oscillation wave component by a flow oscillation wave component is performed for each frequency that was left by the frequency-culling.

2.  The respiratory impedance measuring apparatus of claim 1, wherein
    the control means (31) is configured to cause the air vibration pressure by the oscillation wave, which has only n/T (n: an integer, T: a real number) frequency components to be generated by a pulse series having a cycle T to provide for the frequency-culling.

3.  The respiratory impedance measuring apparatus of claim 1, wherein
    the control means (31) is configured to cause the air vibration pressure by the oscillation wave to be generated by obtaining the oscillation wave that is frequency-culled by combining a plurality of sine waves at a plurality of different frequencies.

4.  The respiratory impedance measuring apparatus of any one of claims 1 to 3, wherein
    the control means (31) comprises a signal input means (35) that is configured to supply an input signal to the pressurizing means (21) such that an oscillation wave having a desired pressure waveform is an output signal, and wherein the input signal is based on reverse computation using an input signal and an output signal of the pressurizing means and a transfer function of the pressurizing means.

5.  The respiratory impedance measuring apparatus of claim 4, wherein
    the signal input means (35) is configured to supply to the pressurizing means (21) as an input signal a signal obtained by adding a specific value to each frequency component of the signal obtained by the reverse computing, or by reverse computing the signal formed by adding an impulse to an onset portion of the output signal.

**6.** The respiratory impedance measuring apparatus of any one of the preceding claims, wherein
the Fourier-transforming means (32) comprises a CIC filter (36) configured to separate prior the Fourier-transforming a breathing signal and an oscillation component for each of the signals obtained by the pressure detecting means (12) and the flow detecting means (13) and
the pressure spectrum and the flow spectrum are obtained based on the respective oscillation component.

**7.** A respiratory impedance measurement method comprising:

a pressurizing step to apply an air vibration pressure to an inside of an oral cavity;
a control step of causing the air vibration pressure to be generated by an oscillation wave, wherein the oscillation wave is a signal that controls the pressurizing step;
a pressure detecting step of detecting a pressure of the inside of the oral cavity;
a flow detecting step of detecting the flow generated by breathing;
a Fourier-transforming step of obtaining and Fourier-transforming signals obtained at the pressure detecting step and the flow detecting step under the pressurized condition provided at the pressurizing step, thereby, obtaining as a result a pressure spectrum for the signal obtained by the pressure detecting step and a flow spectrum for the signal obtained by the flow detecting step; and
a computing step of dividing a pressure oscillation wave component by a flow oscillation wave component; wherein
each of the steps is executed by processing and control
of a computer, **characterised in that** the oscillation wave is obtained by frequency-culling, and the frequency-culling is executed such that the signal has only those frequency components from a plurality of different frequencies that are left after the culling is executed;, and that the method further comprises
an extracting step of
obtaining, for each of the pressure spectrum and the flow spectrum, a breathing high frequency component based those frequency components of the pressure spectrum and the flow spectrum, respectively, that were culled during the frequency-culling,
obtaining a culled pressure spectrum and a culled flow spectrum, each comprising frequency components of the pressure spectrum and the flow spectrum, respectively, that were left by the frequency-culling, and
extracting pressure oscillation wave components and flow oscillation wave components by subtracting the breathing high frequency component from culled pressure spectrum and the culled flow spectrum, respectively; and, that the computing step of dividing is performed for each frequency that was left by the frequency-culling.

**8.** The respiratory impedance measurement method of claim 7, wherein
at the control step, the air vibration pressure by an oscillation wave, which has only $n/T$ (n: an integer, T: a real number) frequency components is caused to be generated by supplying a pulse having a cycle T to provide for the frequency-culling.

**9.** The respiratory impedance measurement method of claim 7, wherein
at the control step, the air vibration pressure by an oscillation wave is caused to be generated by obtaining the oscillation wave, which is frequency-culled, by combining a plurality of sine waves at a plurality of different frequencies.

**10.** The respiratory impedance measurement method of any one of claims 7 to 9, wherein
the control step comprises a signal input step of supplying an input signal at the pressurizing step such that an oscillation wave having a desired pressure waveform is an output signal, and wherein the input signal is based on reverse computation using an input signal and an output signal at the pressurizing step and a transfer function at the pressurizing step.

**11.** The respiratory impedance measurement method of claim 10, wherein
at the signal input step, a signal is supplied at the pressurizing step, that is obtained by adding a specific value to each frequency component of the signal obtained by the reverse computing, or by reverse computing the signal formed by adding an impulse to an onset portion of the output signal.

**12.** A respiratory impedance display method of executing display on a displaying apparatus based on respiratory impedance measured by a respiratory impedance measuring apparatus, the method comprising:

performing the method of any one of claims 7 to 11, thereby measuring an respiratory impedance, wherein the display is executed by three-dimensionally taking values based on an impedance axis, a frequency axis, and a time axis, and wherein
an image is created by including respiratory impedance obtained by executing an interpolation process for culled frequencies, in an image to execute the display by three-dimensionally taking values and, thereby, the display is executed.

13. The respiratory impedance display method of claim 12, wherein
the display is executed by creating an image with the length in the direction of the time axis, that is taken to be a length enough to repeat therein at least two sets of exhalation and inhalation.

14. The respiratory impedance display method of claim 12 or 13, wherein
the display is executed by creating an image that expresses magnitude of an impedance value using variation in color or variation in gradation.

**Patentansprüche**

1. Atemwiderstandsmessvorrichtung mit
einem Druckmittel (21), das zum Anwenden eines Luftvibrationsdrucks im Inneren einer Mundhöhle ausgebildet ist,
einem Steuerungsmittel (30), das zum Veranlassen der Erzeugung des Luftvibrationsdrucks durch eine Oszillationswelle ausgebildet ist, wobei die Oszillationswelle ein Signal ist, das das Druckmittel (21) antreibt,
einem Druckerkennungsmittel (12), das zum Erkennen des Drucks des Inneren der Mundhöhle ausgebildet ist,
einem Strömungserkennungsmittel (13), das zum Erkennen einer durch Atmen erzeugten Strömung ausgebildet ist,
einem Fourier-Transformationsmittel (32), das dazu ausgebildet ist, Signale, die von dem Druckerkennungsmittel (12) und dem Strömungserkennungsmittel (13) unter einer von dem Druckmittel (21) bereitgestellten Druckbedingungen erhalten wurden, zu erhalten und zu Fourier-transformieren, wodurch als ein Ergebnis ein Druckspektrum für das von dem Druckerkennungsmittel (12) erhaltene Signal und ein Strömungsspektrum für das von dem Strömungserkennungsmittel (13) erhaltene Signal erhalten wird,
einem Extraktionsmittel (33) und
einem Berechnungsmittel (34), das zum Teilen einer Druckoszillationswellenkomponente durch eine Strömungsoszillationswellenkomponente ausgebildet ist,
**dadurch gekennzeichnet, dass**
die Oszillationswelle durch Frequenzauswählen erhalten wird und das Frequenzauswählen derart ausgebildet ist, dass das Signal nur diejenigen Frequenzkomponenten von einer Mehrzahl von unterschiedlichen Frequenzen aufweist, die nach dem Auswählen verblieben sind, und
die Extraktionsmittel dazu ausgebildet sind, für das Druckspektrum und das Strömungsspektrum eine Atemhochfrequenzkomponente basierend auf den Frequenzkomponenten des Druckspektrums und des Strömungsspektrums, die während der Frequenzauswahl ausgewählt wurden, zu erhalten, und
das Extraktionsmittel ausgebildet ist,
für das Druckspektrum und das Strömungsspektrum eine Atemhochfrequenzkomponente basierend jeweils auf den Frequenzkomponenten des Druckspektrums und des Strömungsspektrums, die während des Frequenzauswählens ausgewählt wurden, zu erhalten,
ein Auswahldruckspektrum und ein Auswahlströmungsspektrum zu erhalten, die jeweils Frequenzkomponenten des Druckspektrums und des Strömungsspektrums aufweisen, die durch die Frequenzauswahl verblieben sind, und Druckoszillationswellenkomponenten und Strömungsoszillationswellenkomponenten durch Subtraktion der Atemhochfrequenzkomponente von dem Auswahldruckspektrum und dem Auswahlströmungsspektrum zu extrahieren und die Teilung einer Druckoszillationswellenkomponente durch eine Strömungsoszillationswellenkomponente für jede Frequenz, die durch die Frequenzauswahl verblieben ist, auszuführen.

2. Atemwiderstandsmessvorrichtung nach Anspruch 1, wobei das Steuerungsmittel (31) zum Verursachen, dass der Luftvibrationsdruck von der Oszillationswelle, die nur n/T (n: ganze Zahl, T: reale Zahl) Frequenzkomponenten hat, durch eine Pulsserie mit einem Zyklus T zur Bereitstellung für die Frequenzauswahl erzeugt wird, ausgebildet ist.

3. Atemwiderstandsmessvorrichtung nach Anspruch 1, wobei die Steuerungsmittel (31) zum Verursachen, dass der Luftvibrationsdruck von der Oszillationswelle erzeugt wird durch Erhalten einer Oszillationswelle, die frequenzausgewählt ist, durch Kombination einer Mehrzahl von Sinuswellen bei einer Mehrzahl von unterschiedlichen Frequenzen, ausgebildet ist.

4. Atemwiderstandsmessvorrichtung nach einem der Ansprüche 1 bis 3, wobei die Steuerungsmittel (31) ein Signaleingangsmittel (35) aufweisen, das dazu ausgebildet ist, ein Eingangssignal für das Druckmittel (21) bereitzustellen, so dass eine Oszillationswelle mit einer gewünschten Druckwellenform ein Ausgangssignal ist, und wobei das Eingangssignal auf Rückberechnung unter Benutzung eines Eingangssignals und eines Ausgangssignals des Druckmittels und einer Übertragungsfunktion des Druckmittels basiert.

5. Atemwiderstandsmessvorrichtung nach Anspruch 4, wobei das Signaleingangsmittel (35) zum Versorgen des Druckmittels (21) als Eingangssignal mit einem Signal, welches durch Addieren eines spezifischen Wertes zu jeder Frequenzkomponente des Signals, welches durch Rückberechnung erhalten wurde, oder doch Rückberechnung des Signals, welches durch Addieren eines Impulses zu einem Anfangsabschnitt des Ausgangssignals gebildet wurde, erhalten wird, ausgebildet ist.

6. Atemwiderstandsmessvorrichtung nach einem der vorhergehenden Ansprüche, wobei die Fourier-Transformationsmittel (32) ein CIC-Filter (36) aufweisen, das dazu ausgebildet ist, vor der Fourier-Transformation ein Atemsignal und eine Oszillationskomponente für jede der Signale, die durch das Druckerkennungsmittel (12) und das Strömungserkennungsmittel (13) erhalten wurden, abzutrennen und
das Druckspektrum und das Strömungsspektrum basierend auf der entsprechenden Oszillationskomponente zu erhalten.

7. Atemwiderstandsmessverfahren mit
einem Druckschritt zum Anwenden eines Luftvibrationsdrucks an ein Inneres einer Mundhöhle,
einem Steuerungsschritt zum Verursachen, dass der Luftvibrationsdruck mit einer Oszillationswelle erzeugt wird, wobei die Oszillationswelle ein Signal ist, welches den Druckschritt steuert,
einem Druckerkennungsschritt zum Erkennen eines Drucks des Inneren der Mundhöhle, einem Strömungserkennungsschritt zum Erkennen der durch Atmung erzeugten Strömung,
einem Fourier-Transformationsschritt zum Erhalten und Fourier-Transformieren von Signalen, die unter der beim Druckschritt vorgesehenen Druckbedingung beim Druckerkennungsschritt und Strömungserkennungsschritt erhalten wurden, wodurch als Ergebnis ein Druckspektrum für das im Druckerkennungsschritt erkannte Signal und ein Strömungsspektrum für das im Strömungserkennungsschritt erkannte Signal erhalten wird, und
einem Berechnungsschritt des Teilens einer Druckoszillationswellenkomponente durch eine Strömungsoszillationswellenkomponente, wobei
jeder der Schritte durch Verarbeiten und Steuern eines Computers ausgeführt wird, **dadurch gekennzeichnet, dass** die Oszillationswelle durch Frequenzauswahl erhalten wird und die Frequenzauswahl derart ausgeführt wird, dass das Signal nur diejenigen Frequenzkomponenten von einer Mehrzahl von unterschiedlichen Frequenzen, die nach der Auswahl verblieben sind, hat, und dass das Verfahren ferner aufweist
einen Extraktionsschritt, bei dem
für das Druckspektrum und das Strömungsspektrum eine Atemhöchfrequenzkomponente basierend jeweils auf denjenigen Frequenzkomponenten des Druckspektrums und des Strömungsspektrums, die während der Frequenzauswahl ausgewählt werden, erhalten wird,
ein Auswahldruckspektrum und ein Auswahlströmungsspektrum, die jeweils Frequenzkomponenten des Druckspektrums und des Strömungsspektrums, die jeweils von der Frequenzauswahl verblieben sind, aufweisen, erhalten werden und
Druckoszillationswellenkomponenten und Strömungsoszillationswellenkomponenten jeweils durch Subtrahieren der Atemhochfrequenzkomponente von dem Auswahldruckspektrum und dem Auswahlströmungsspektrum erhalten werden, und
dass der Berechnungsschritt des Teilens für jede Frequenz, die bei der Frequenzauswahl verblieben ist, durchgeführt wird.

8. Atemwiderstandsmessverfahren nach Anspruch 7, wobei beim Steuerungsschritt verursacht wird, dass der Luftvibrationsdruck von einer Oszillationswelle, die nur $n/T$ (n: ganze Zahl, T: reale Zahl) Frequenzkomponenten hat, durch Versorgen eines Pulses mit einem Zyklus T zum Vorsehen der Frequenzauswahl erzeugt wird.

9. Atemwiderstandsmessverfahren nach Anspruch 7, wobei im Steuerungsschritt die Erzeugung des Luftvibrationsdrucks von einer Oszillationswelle verursacht wird durch Erhalten der Oszillationswelle, welche frequenzausgewählt ist, durch Kombinieren einer Mehrzahl von Sinuswellen bei einer Mehrzahl von unterschiedlichen Frequenzen.

10. Atemwiderstandsmessverfahren nach einem der Ansprüche 7 bis 9, wobei der Steuerungsschritt einen Signaleingabeschritt aufweist, beim dem ein Eingangssignal beim Druckschritt derart bereitgestellt wird, dass eine Oszillationswelle mit einer gewünschten Druckwellenform ein Ausgangssignal ist, und wobei das Eingangssignal auf Rückberechnung unter Benutzung eines Eingangssignals und eines Ausgangssignals beim Druckschritt und einer Transferfunktion beim Druckschritt basiert.

11. Atemwiderstandsmessverfahren nach Anspruch 10, wobei beim Signaleingabeschritt ein Signal beim Druckschritt bereitgestellt wird, das durch Addieren eines spezifischen Werts zu jeder Frequenzkomponente des durch Rückberechnung erhaltenen Signals oder durch Rückberechnung des durch Addieren eines Impulses zu einem Anfangsabschnitt des Ausgangssignals geformten Signals erhalten wird.

12. Atemwiderstandsdarstellverfahren zum Darstellen auf einer Darstellvorrichtung basierend auf einem Atemwiderstand, der durch eine Atemwiderstandsmessvorrichtung gemessen wird, mit
Ausführen des Verfahrens nach einem der Ansprüche 7 bis 11, wodurch ein Atemwiderstand gemessen wird, wobei das Darstellen durch dreidimensionales Nehmen von Werten basierend auf einer Widerstandsachse, einer Frequenzachse und einer Zeitachse ausgeführt wird und wobei
ein Bild durch Aufnehmen eines Atemwiderstands erzeugt wird, welcher durch Ausführen eines Interpolationsprozesses für ausgewählte Frequenzen erhalten wird, in einem Bild um ein Darstellen durch dreidimensionales Nehmen von Werten auszuführen und dadurch das Darstellen auszuführen.

13. Atemwiderstandsdarstellverfahren nach Anspruch 12, wobei das Darstellen durch Erzeugen eines Bildes mit der Länge in Richtung der Zeitachse, die als eine Länge genommen wird, die ausreicht darin mindestens zwei Sätze von Ausatmen und Einatmen zu wiederholen, ausgeführt wird.

14. Atemwiderstandsdarstellverfahren nach Anspruch 12 oder 13, wobei das Darstellen durch Erzeugen eines Bildes, das eine Größe eines Widerstandswertes unter Benutzung von Farbvariationen oder Abstufungsvariationen ausdrückt, ausgeführt wird.

**Revendications**

1. Appareil de mesure d'impédance respiratoire comprenant:

un moyen de pressurisation (21) qui est configuré pour appliquer une pression de vibration de l'air à un intérieur d'une cavité buccale;
un moyen de contrôle (30) qui est configuré pour provoquer la pression de vibration de l'air à être générée par une onde d'oscillation, dans lequel l'onde d'oscillation est un signal qui entraîne le moyen de pressurisation (21) ;
un moyen de détection de pression (12) qui est configuré pour détecter une pression de l'intérieur de la cavité buccale;
un moyen de détection de flux (13) qui est configuré pour détecter un flux généré par la respiration ;
un moyen de transformation de Fourier (32) qui est configuré pour obtenir des signaux par transformation de Fourier obtenus par le moyen de détection de pression (12) and le moyen de détection de flux (13) sous une condition de pressurisation fournie par le moyen de pressurisation (21), obtenant ainsi comme résultat un spectre de pression pour le signal obtenu par le moyen de détection de pression (12) et un spectre de flux pour le signal obtenu par le moyen de détection de flux (13) ;
un moyen d'extraction (33) ; et
un moyen de calcul (34) qui est configuré pour diviser une composante d'onde d'oscillation de pression par une composante d'onde d'oscillation de flux ;

**caractérisé en ce que** :

l'onde d'oscillation est obtenue par triage de fréquences, et le triage de fréquences est configurée de telle sorte que le signal ne comporte que ces composantes de fréquence d'une pluralité de différentes fréquences qui sont laissées après l'exécution du triage et ;
le moyen d'extraction est configuré pour obtenir, pour chacun des spectres de pression et des spectres de flux, une composante haute fréquence de respiration basée sur ces composantes de fréquence du spectre de pression et du spectre de flux, respectivement, qui étaient triés pendant le triage de fréquences,
pour obtenir un spectre de pression trié et un spectre de flux trié, chacun comprenant des composantes de fréquence comprise dans le spectre de fréquence et dans le spectre de flux, respectivement, qui étaient laissées par le triage de fréquences, et
pour extraire des composantes d'onde d'oscillation de pression et des composantes d'onde d'oscillation de flux en soustrayant la composante haute fréquence de respiration du spectre de pression trié et du spectre de flux trié, respectivement; et

la division d'une composante d'onde d'oscillation de pression par une composante d'onde d'oscillation de flux est effectuée pour chaque fréquence qui était laissée par le triage de fréquences.

2. Appareil de mesure d'impédance respiratoire selon la revendication 1, dans lequel
le moyen de contrôle (31) est configuré pour provoquer la pression de vibration de l'air par l'onde d'oscillation, qui a seulement n/T (n : un entier, T : un nombre réel) composantes de fréquence, à être générée par une série d'impulsions ayant une période T à fournir pour le triage de fréquences.

3. Appareil de mesure d'impédance respiratoire selon la revendication 1, dans lequel
le moyen de contrôle (31) est configuré à provoquer la pression de vibration de l'air par l'onde d'oscillation à être générée en obtenant l'onde d'oscillation, dont la fréquence est triée, en combinant une pluralité d'ondes sinusoïdales à une pluralité de différentes fréquences.

4. Appareil de mesure d'impédance respiratoire selon l'une quelconque des revendications 1 à 3, dans lequel
le moyen de contrôle (31) comprend un moyen d'entrée de signal (35) qui est configuré pour fournir un signal d'entrée au moyen de pressurisation (12) de telle sorte qu'une onde d'oscillation ayant une forme d'onde de pression désirée est un signal de sortie, et dans lequel le signal d'entrée est basé sur un calcul inverse utilisant un signal d'entrée et un signal de sortie du moyen de pressurisation et une fonction de transfert du moyen de pressurisation.

5. Appareil de mesure d'impédance respiratoire selon la revendication 4, dans lequel
le moyen d'entrée de signal (35) est configuré pour fournir au moyen de pressurisation (21) comme signal d'entrée un signal obtenu en additionnant une valeur spécifique à chaque composante de fréquence du signal obtenue par le calcul inverse, ou par le calcul inverse du signal formé en additionnant une impulsion à une partie du début du signal de sortie.

6. Appareil de mesure d'impédance respiratoire selon l'une quelconque des revendications précédentes, dans lequel
le moyen de transformation de Fourier (32) comprend un filtre CIC (36) configuré pour séparer avant la transformation de Fourier un signal de respiration et une composante d'oscillation pour chacun des signaux obtenus par les moyens de détection de pression (12) et les moyens de détection de flux (13) et
le spectre de pression et le spectre de flux sont obtenus basés sur la composante d'oscillation respective.

7. Méthode de mesure d'impédance respiratoire comprenant :

une étape de pressurisation pour appliquer une pression de vibration de l'air à un intérieur d'une cavité buccale ;
une étape de contrôle provoquant la pression de vibration de l'air à être générée par une onde d'oscillation, dans laquelle l'onde d'oscillation est un signal qui contrôle l'étape de pressurisation ;
une étape de détection de pression pour détecter une pression de l'intérieur de la cavité buccale ;
une étape de détection de flux pour détecter le flux généré par la respiration ;
une étape de transformation de Fourier pour obtenir des signaux par transformation de Fourier obtenus à l'étape de détection de pression et l'étape de détection de flux sous la condition de pressurisation fournie à l'étape de pressurisation, ainsi, obtenir comme résultat un spectre de pression pour le signal obtenu par l'étape de détection de pression et un spectre de flux pour le signal obtenu par l'étape de détection de flux ; et
une étape de calcul de division d'une composante d'onde d'oscillation de pression par une composante d'onde d'oscillation de flux; dans laquelle
chacune des étapes est exécutée par traitement et contrôle d'un ordinateur, **caractérisé en ce que** l'onde d'oscillation est obtenue par sélection de fréquences, et le triage de fréquences est exécutée de telle sorte que le signal ne comporte que les composantes de fréquence d'une pluralité de différentes fréquences qui sont laissées après que le triage est exécutée, et que la méthode comprenne en outre
une étape d'extraction de
l'obtention, pour chacun des spectres de pression et des spectres de flux, une composante haute fréquence de respiration basée sur ces composantes de fréquence du spectre de pression et du spectre de flux, respectivement, qui étaient triées pendant le triage de fréquences,
l'obtention d'un spectre de pression trié et d'un spectre de flux trié, chaque composante de fréquence comprise dans le spectre de fréquence et dans le spectre de flux, respectivement, qui étaient laissées par le triage de fréquences, et
l'extraction des composantes d'onde d'oscillation de pression et des composantes d'onde d'oscillation de flux en soustrayant la composante haute fréquence de respiration du spectre de pression trié et du spectre de flux trié, respectivement ; et,

et que l'étape de calcul de division est effectuée pour chaque fréquence qui était laissée par le triage de fréquences.

8. Méthode de mesure d'impédance respiratoire selon la revendication 7, dans laquelle
à l'étape de contrôle, la pression de vibration de l'air par une onde d'oscillation, qui a seulement n/T (n : un entier, T : un nombre réel) composantes de fréquence, est provoquée à être générée en fournissant une impulsion ayant une période T à fournir pour le triage de fréquences.

9. Méthode de mesure d'impédance respiratoire selon la revendication 7, dans laquelle
à l'étape de contrôle, la pression de vibration de l'air par une onde d'oscillation est provoquée à être générée en obtenant l'onde d'oscillation, dont la fréquence est triée, en combinant une pluralité d'ondes sinusoïdales à une pluralité de différentes fréquences.

10. Méthode de mesure d'impédance respiratoire selon l'une quelconque des revendications 7 à 9, dans laquelle
l'étape de contrôle comprend une étape d'entrée du signal pour fournir un signal d'entrée à l'étape de pressurisation de telle sorte qu'une onde d'oscillation ayant une forme d'onde de pression désirée est un signal de sortie, et dans lequel le signal d'entrée est basé sur un calcul inverse utilisant un signal d'entrée et un signal de sortie de l'étape de pressurisation et une fonction de transfert de l'étape de pressurisation.

11. Méthode de mesure d'impédance respiratoire selon la revendication 10, dans laquelle
à l'étape d'entrée de signal, un signal est fourni à l'étape de pressurisation, qui est obtenue en additionnant une valeur spécifique à chaque composante de fréquence du signal obtenu par le calcul inverse, ou par le calcul inverse du signal formé en additionnant une impulsion à une partie du début du signal de sortie.

12. Méthode d'affichage d'impédance respiratoire d'exécution d'affichage sur un appareil d'affichage basé sur l'impédance respiratoire mesurée par un appareil de mesure d'impédance respiratoire, la méthode comprenant :

l'exécution de la méthode d'une quelconque des revendications 7 à 11, en mesurant une impédance respiratoire, dans laquelle
l'affichage est exécuté en prenant des valeurs tridimensionnelles basées sur un axe d'impédance, un axe de fréquence, et un axe de temps, et dans lequel
une image est créée en incluant l'impédance respiratoire obtenue en exécutant un processus d'interpolation pour les fréquences triées, dans une image pour exécuter l'affichage en prenant des valeurs tridimensionnelles et, ainsi, l'affichage est exécuté.

13. Méthode d'affichage d'impédance respiratoire selon la revendication 12, dans laquelle
l'affichage est exécuté en créant une image avec la longueur dans la direction de l'axe du temps, qui est prise à être une longueur assez pour y répéter au moins deux séries d'exhalation et l'inhalation.

14. Procédé d'affichage d'impédance respiratoire selon la revendication 12 ou 13, dans laquelle
l'affichage est exécuté par la création d'une image qui exprime l'amplitude d'une valeur d'impédance en utilisant une variation de couleur ou une variation en gradation.

F I G 1

F I G 2

(a

25m s

(b)

0.5sec | 0.5sec

F I G 3

25m s

F I G 4

F I G 5

( a )

(b)

(c) $\underrightarrow{X\ (\ \omega)}$ | H ( ω) | $\underrightarrow{Y\ (\ \omega)}$

(d)

(e)

F I G 6

(a)

(b)

3 6

CIC FILTER

STARTING
POSITION

1s

0.5s

0.5s

F I G 7

H z

F I G 8

A HANNING WINDOW IS SET.

1s

F I G 9

3 2

FFT

OSCILLATION WAVE
+BREATHING
SIGNAL SPECTRUM

BREATHING
SIGNAL

1  2  3  4  5  6

F I G 1 2

RESISTANCE

REACTANCE

F I G 1 3

( a )

3 6

CIC FILTER

( b )

FIG14

(a)

1-SECOND SECTION IS TAKEN

1s

3 2

FFT

(b)

OSCILLATION
WAVE
+BREATHING

BREATHING
SIGNAL

1  2  3  4  5  6

FIG15

HEALTHY PERSON(66yrs)

COPD PATIENT (66yrs;%FEV1=24.4)

Rrs

TIME

Frequency
(3~35Hz)

EXHALATION    EXHALATION    EXHALATION

INHALATION    INHALATION    INHALATION

(a)

(b)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6142952 A
- US 6435182 B1
- US 2005247307 A1
- JP H03039140 B

### Non-patent literature cited in the description

- Use Of Transfer Impedance Measurements For Clinical Assessment of Lung Mechanics. **LUTCHEN K R et al.** AMERICAN JOURNAL OF RESPIRATORY AND CRITICAL CARE MEDICINE. AMERICAN LUNG ASSOCIATION, 01 January 1998, vol. 157, 435-446
- **IONESCU C et al.** Detection of novel respiratory mechanic parameters by means of forced oscillations. *ROCEEDINGS OF THE WORLD CONGRESS OF IFAC,* 04 July 2005, vol. XX, XX (16TH), 1-6
- The forced oscillation technique in clinical practice: methodology, recommendations and future developments. **OOSTVEN E et al.** EUROPEAN RESPIRATORY JOURNAL. MUNKSGAARD INTERNATIONAL PUBLISHERS, 01 January 2003, vol. 22, 1026-1041